## Europäisches Patentamt

## European Patent Office

## Office européen des brevets

(11) Veröffentlichungsnummer: **0 164 439**
**B1**

(12) **EUROPÄISCHE PATENTSCHRIFT**

(45) Veröffentlichungstag der Patentschrift:
**29.06.88**

(51) Int. Cl.⁴: **A 61 B 3/00**

(21) Anmeldenummer: **84107848.8**

(22) Anmeldetag: **05.07.84**

(54) Übungs- und Untersuchungsgerät für die menschlichen Augen.

(30) Priorität: **07.06.84 DE 3421132**

(43) Veröffentlichungstag der Anmeldung:
**18.12.85 Patentblatt 85/51**

(45) Bekanntmachung des Hinweises auf die Patenterteilung:
**29.06.88 Patentblatt 88/26**

(84) Benannte Vertragsstaaten:
**AT BE CH DE FR GB IT LI LU NL SE**

(56) Entgegenhaltungen:
**DE-B-1 132 744**
**US-A-2 246 687**
**US-A-2 277 122**
**US-A-2 893 288**
**US-A-3 545 847**
**US-A-4 128 317**

(73) Patentinhaber: **OCUTEC- Vertriebsgesellschaft medizinischer Geräte mbH, Otto- Brenner-Strasse 122, D-4800 Bielefeld 1 (DE)**

(72) Erfinder: **Schröter, Hans, Oberer Steinweg 16, D-4803 Steinhagen/Westf. (DE)**

(74) Vertreter: **Patentanwälte TER MEER - MÜLLER - STEINMEISTER, Artur- Ladebeck- Strasse 51, D-4800 Bielefeld 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

## Beschreibung

Die Erfindung betrifft ein Übungs- und Untersuchungsgerät für die menschlichen Augen mit einem an einem Ende eines langgestreckten Gehäuses aufragenden Rahmen, der eine Stirn- und/oder Kinnstütze sowie eine Halterung für zwei optische Gläser trägt, und einer in Längsrichtung des Gehäuses verfahrbaren, im wesentlichen in Höhe der optischen Gläser liegenden Lichtquelle am oberen Ende eines Stabes, der aus einem Schlitz des Gehäuses austritt und durch eine im Gehäuse liegende, durch einen Motor in Drehung versetzbare, in Längsrichtung des Gehäuses verlaufende Spindel antreibbar ist.

Ein derartiges Gerät ist aus der DE-U-7 905 160 bekannt. Soweit dieser, im wesentlichen auf eine Prinzipdarstellung des beschriebenen Geräts beschränkten Druckschrift zu entnehmen ist, wird die Spindel durch einen umsteuerbaren Servomotor alternativ in entgegengesetzte Richtung angetrieben, während sich auf der Spindel eine Mutter befindet, die unmittelbar einen Stab mit am oberen Ende liegender Lichtquelle trägt. Eine zusätzliche Führung für die Mutter und den Stab ist offenbar nicht vorgesehen, sofern man davon absieht, daß der Stab in einem Schlitz des zugehörigen Gehäuses läuft, der zumindest eine gewisse Abstützung senkrecht zur Bewegungsrichtung gewährleisten dürfte. Der Stab ist offenbar fest auf der Mutter angebracht, folgt also deren Kippbewegungen um die Spindelachse.

Der Erfindung liegt die Aufgabe zugrunde, ein vielseitig verwendbares und zugleich in seinem Aufbau und seiner Bedienbarkeit verhältnismäßig einfaches Gerät der eingangs genannten Art zu schaffen.

Diese Aufgabe wird erfindungsgemäß dadurch gelöst, daß im oberen Bereich des Gehäuses zwei in dessen Längsrichtung verlaufende Führungsachsen vorgesehen sind, auf denen ein Schlitten, der den Stab trägt, gleitend verschiebbar ist, und daß der Schlitten an einem schwenkbaren Arm ein Mutternsegment trägt, das durch Schwenken des Armes mit der Spindel in Eingriff bringbar ist.

Vorzugsweise ist der Stab an einem Abdeckband befestigt, das breiter als der Schlitz ist, und zu dessen Führung zwei dessen seitliche Ränder übergreifende Leisten an der Oberseite des Gehäuses befestigt sind.

Entsprechend einer bevorzugten Ausführungsform ist angrenzend an den Stab eine Druckplatte vorgesehen, die über einen in senkrechter Richtung verschiebbaren Druckstab derart mit dem Mutternsegment in Verbindung steht, daß durch Niederdrücken der Druckplatte das Mutternsegment aus dem Gewinde der Spindel abhebbar ist.

Das erfindungsgemäße Gerät ermöglicht die Untersuchung aller für das beidäugige Sehen wesentlichen Teilfunktionen, nämlich die Akkomodation, die Vergenz und die motorische und sensorische Fusion. Bei Störungen in der Zusammenarbeit der Augen können Übungen unter Berücksichtigung der einzelnen Teilfunktionen durchgeführt werden. Das Blickfeld in den freien Raum ist nicht beeinträchtigt. Durch Schweifgläser (Bagoliniglaser) kann die Fusion der Augen aufgehoben werden, so daß beide Augen gesondert kontrolliert werden können.

Im folgenden werden bevorzugte Ausführungsbeispiele der Erfindung anhand der beigefügten Zeichnung näher erläutert.

Fig. 1 ist eine perspektivische Gesamtdarstellung einer Ausführungsform eines erfindungsgemäßen Gerätes;

Fig. 2 ist eine Ansicht von einer Stirnseite des Gerätes her;

Fig. 3 zeigt eine Seitenansicht zu Fig. 2;

Fig. 4 ist eine teilweise aufgeschnittene Draufsicht zu Fig. 3;

Fig. 5 ist ein Querschnitt durch das Gehäuse des Gerätes;

Fig. 6 ist ein zugehöriger Längsschnitt;

Fig. 7 veranschaulicht die Befestigung des die Lichtquelle tragenden Stabes.

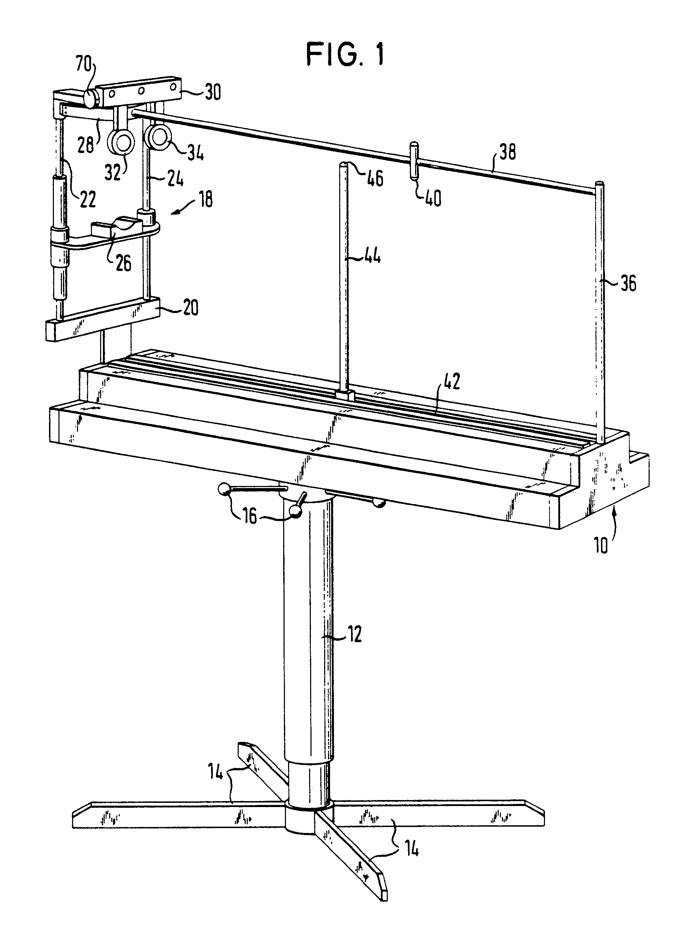

Ein in Fig. 1 gezeigtes erfindungsgemäßes Gerät umfaßt ein langgestrecktes, kastenförmiges Gehäuse 10, das auf einer Säule 12 angeordnet ist. Am unteren Ende der Säule 12 sind Füße 14 vorgesehen. Am oberen Ende der Säule sind eine Anzahl von sternförmig angeordneten Handgriffen erkennbar, die eine Höhenverstellung der Säule ermöglichen. Im übrigen ist das Gehäuse 10 in nicht gezeigter Weise von der Säule 12 abnehmbar, so daß es als Tischgerät benutzt werden kann.

An dem links in Fig. 1 liegenden Ende des Gehäuses 10 befindet sich ein aufragender Rahmen 18, der ein T-förmigen Fuß 20 umfaßt, von dessen beiden Enden senkrechte Führungsstäbe 22,24 ausgehen. Die Führungsstäbe 22,24 werden überbrückt durch eine höhenverstellbare Kinnstütze 26 und - im oberen Bereich - durch eine Stirnstütze 28. Den oberen Abschluß des Rahmens 18 bildet eine Halterung 30, an deren Unterseite zwei Aufnahmefassungen 32,34 für verschiedene optische Gläser, beispielsweise Schweifgläser oder Herschelprismen angeordnet sind. Der Abstand der Aufnahmefassungen 32,34 ist verstellbar.

Am rechten Ende des Gehäuses 10 ist eine senkrecht aufragende Stütze 36 befestigt, deren oberes Ende durch einen Längsstab 38 mit dem Rahmen 18 verbunden ist. Auf dem Längsstab 38 ist ein längs verschiebbarer und festlegbarer Fixierpunkt 40 geführt.

Das Gehäuse 10 weist auf seiner Oberseite einen linksgerichteten Schlitz 42 auf, in dem das untere Ende eines senkrecht aufragenden Stabs 44 angeordnet ist. Der Stab 44 ist durch einen in Figur 1 nicht gezeigten Antrieb in Längsrichtung des Gehäuses 10 hin- und hergehend verfahrbar. Am oberen Ende des Stabes 44 befindet sich eine Lichtquelle 46.

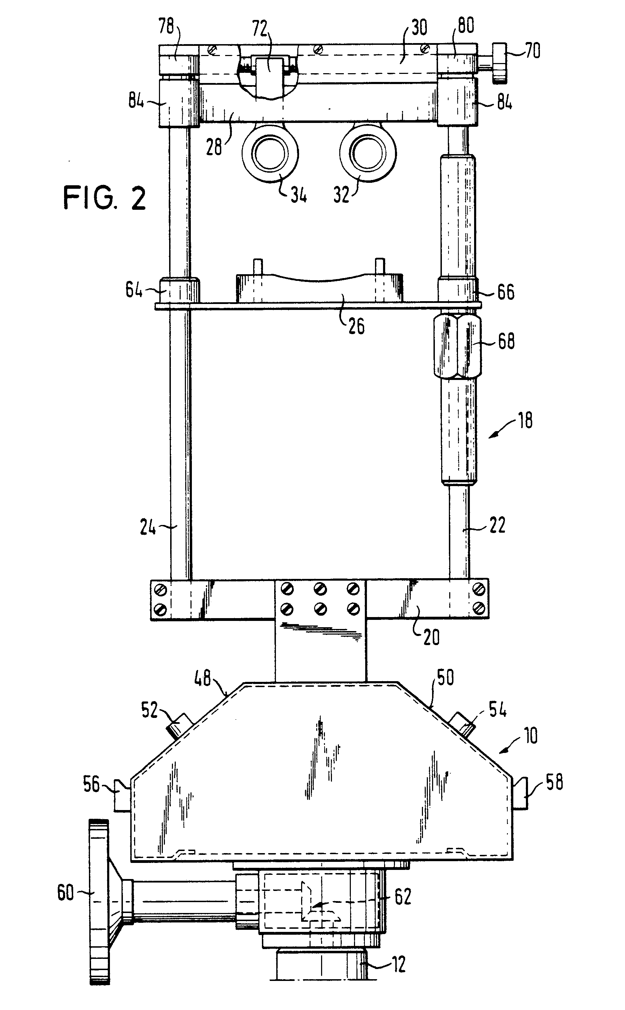

Figur 2 ist eine Stirnansicht des Gehäuses 10 von der Seite des Rahmens 18 her. Im Gegensatz zu Fig. 1 ist das Gehäuse 10 in Fig. 2 an den seitlichen Rändern nicht abgestuft, sondern abgeschrägt. Auf den schrägen Flanken 48,50 befinden sich Druckknöpfe 52,54, mit deren Hilfe der Patient die Lichtquelle 46 vorwärts und rückwärts verfahren kann. Unterhalb der Druckknöpfe befinden sich an den äußeren Seiten des Gehäuses 10 Leisten 56,58 zur Abstützung der Hand.

In Abweichung von Figur 1 erfolgt die Höhenverstellung der Säule 12 bei der Ausführungsform gemäß Figur 2 mit Hilfe eines Kurbelrades 60, dessen Drehung mit Hilfe eines Kegelgetriebes 62 senkrecht nach unten umgelenkt wird.

Die Kinnstütze 26 weist gemäß Fig. 2 an den beiden äußeren Enden Muffen 64,66 auf, die auf den senkrechten Führungsstäben 22,24 verschiebbar sind. Eine Spannhülse 68 dient zur Festlegung der Kinnstütze 26 in gewünschter Höhe.

Wie im oberen Bereich von Fig. 2 zu erkennen ist, befindet sich an einem Ende der Halterung 30 ein Handrad 70. Dieses Handrad 70 ist auf einer nicht im einzelnen gezeigten Spindel befestigt, die mit gegenläufigem Innengewinde von Ansätzen 72 an der oberen Seite der Aufnahmefassungen 32,34 eintritt. Durch Drehung des Handrades 70 kann somit der Abstand der Aufnahmefassungen 32,34 eingestellt werden.

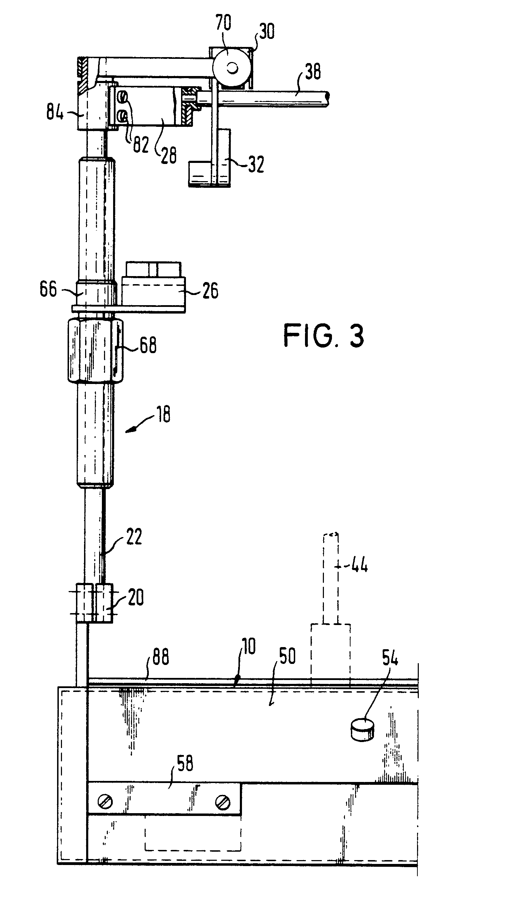

In Fig. 3 ist eine Seitenansicht zu Fig. 2 gezeigt. Zusätzlich ist der verfahrbare Stab gestrichelt angedeutet.

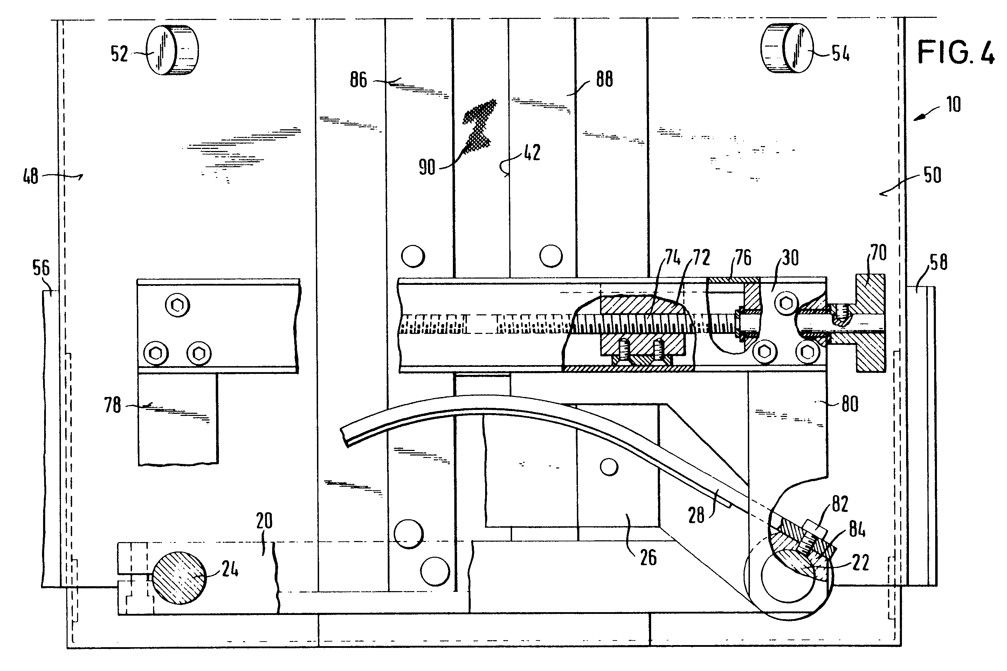

Fig. 4 veranschaulicht in größerem Maßstab Einzelheiten der Halterung 30 für die optischen Gläser. Die bereits erwähnte Spindel ist in diesem Falle mit der Bezugsziffer 74 versehen. Auf der Spindel 74 ist ein Ansatz 72 einer der Aufnahmefassungen 32,34 erkennbar. Die Spindel 74 liegt in einem nach unten offenen Gehäuse 76, das in Fig. 4 lediglich angedeutet ist. Dieses Gehäuse 76 ist über Arme 78,80 mit den senkrechten Führungsstäben 22,24 verbunden. Die Stirnstütze 28 ist an ihren Enden mit Hilfe von Schrauben 82 an Hülsen 84 befestigt, die auf den Führungsstäben 22,24 angebracht sind.

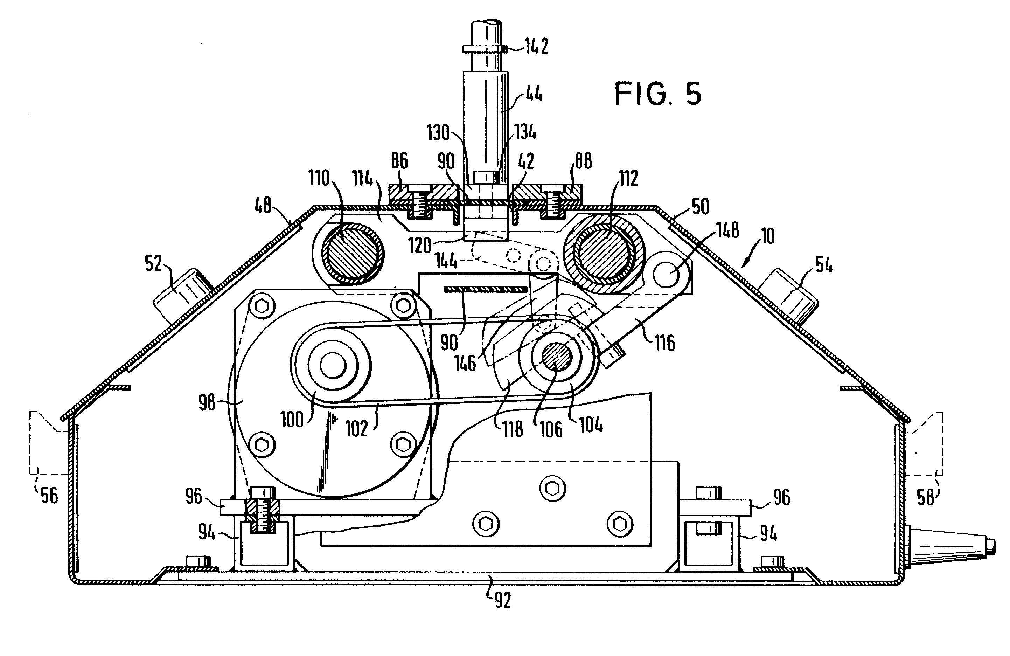

Fig. 5 zeigt einen Querschnitt durch das Gehäuse 10 und läßt einige Einzelheiten des Antriebs der Lichtquelle bzw. des senkrechten Stabes 44 erkennen. Auf der Oberseite des Gehäuses 10 befindet sich in dessen Längsmittellinie der bereits erwähnte Schlitz 42. Beidseitig des Schlitzes 42 sind auf der Oberseite des Gehäuses Leisten 86,88 befestigt. Die Leisten liegen auf ihren einander zugewandten Seiten in Abstand zu der Oberfläche des Gehäuses und bilden insoweit nicht näher bezeichnete Führungsnuten für die Aufnahme der seitlichen Ränder eines Abdeckbandes 90. Auf diesem Abdeckband 90 ist der Stab 44 befestigt. Das Abdeckband 90 deckt den Schlitz 42 über die gesamte Länge des Gehäuses 42 ab und verhindert somit, daß Staub in das Innere des Gehäuses eintreten kann oder eine Verletzungsgefahr durch Hineingreifen in das Gehäuse besteht.

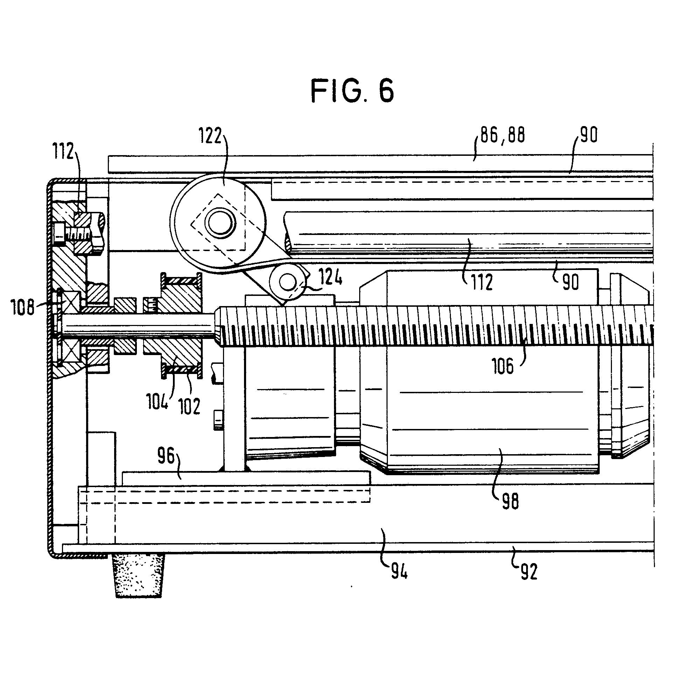

Das Gehäuse 10 besteht insgesamt aus verhältnismäßig dünnem Blech, weist jedoch im Bereich des Bodens eine stärkere Bodenplatte 92 auf, auf der die einzelnen Aggregate innerhalb des Gehäuses befestigt sind. Auf der Bodenplatte 92 ist über Distanzstücke 94 eine Trägerplatte 96 befestigt, die einen Getriebemotor 98 trägt. Auf der nicht bezeichneten Ausgangswelle des Getriebemotors 98 befindet sich eine Riemenscheibe 100, die über einen Riemen 102 eine Riemenscheibe 104 antriebt, die auf einer Spindel 106 befestigt ist. Wie aus Fig. 6 hervorgeht, ist die Spindel 106 in Lagern 108 in den beiden Stirnseiten des Gehäuses gelagert. Die Spindel 106 durchläuft somit das Gehäuse 10 und dient als Antrieb für den Stab 44.

Wie aus Fig. 5 hervorgeht, befinden sich im oberen Bereich des Gehäuses 10 zwei in Längsrichtung des Gehäuses verlaufende Führungsachsen 110,112, auf denen ein Schlitten 114 in Längsrichtung des Gehäuses verschiebbar ist. Am rechten Ende des Schlittens 114 gemäß Fig. 5 befindet sich ein schwenkbarer Arm 116, an dessen freiem Ende ein Mutternsegment 118 befestigt ist, das in Eingriff mit der Spindel 106 gebracht werden kann. Durch diesen Eingriff wird der Schlitten 114 in Längsrichtung des Gehäuses verschoben. Der Schlitten 114 erfaßt einen Ansatz 120 an der Unterseite des oberen Trums des Abdeckbandes 90 unterhalb des senkrechten Stabes 44. Auf diese Weise kann der Stab 44 mit der Lichtquelle 46 im wesentlichen über die gesamte Länge des Gehäuses 10 verfahren werden.

In Fig. 6 ist zusätzlich eine Umlenkrolle 122 und eine Spannrolle 124 für das Abdeckband 90 erkennbar.

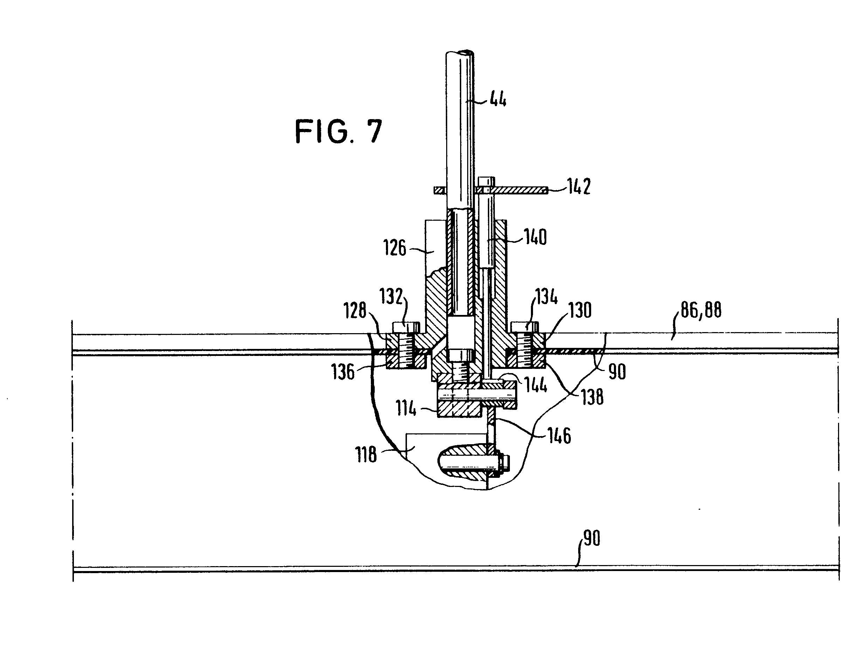

Fig. 7 zeigt die Befestigung des unteren Endes des Stabes 44 an dem Abdeckband 90. Eine Hülse 126 nimmt einerseits in einer nicht bezeichneten Bohrung den Stab 44 auf und besitzt andererseits am unteren Ende zwei seitliche Ansätze 128,130, die mit Hilfe von Schrauben 132,134 und Gegenstücken 136,138 an den Enden des geteilten Förderbandes 90 befestigt sind.

In einer weiteren, nicht bezeichneten, senkrechten Bohrung der Hülse 126 ist ein Druckstab 140 verschiebbar geführt, auf dessen oberem Ende eine Druckplatte 142 befestigt ist, die in einer Bohrung von dem Stab 44 durchlaufen wird. Ein verjüngter, unterer Abschnitt des Druckstabs 140 erstreckt sich nach unten in das Gehäuse 10 hinein und wirkt dort auf einen schwenkbar an dem Schlitten 114 angebrachten Doppelhebel 144 (Fig. 5) ein, dessen gegenüberliegendes Ende schwenkbar mit einer Lasche 146 verbunden ist. Das andere Ende der Lasche 146 steht mit dem bereits erwähnten Mutternsegment 118 derart in Verbindung, daß bei Drehung des Doppelhebels 144 im Gegenuhrzeigersinn das Muttersegment 118 mit dem Arm 116 um seine Schwenkachse 148 auf dem Schlitten 114 angehoben wird. Dabei wird die Antriebsverbindung zwischen der Spindel 106 und dem Muttersegment 118 gelöst.

Wenn daher die Druckplatte 142 niedergedrückt wird, wird der Antrieb des Stabes 44 ausgekuppelt, so daß eine Schnelleinstellung der Position des Stabs vorgenommen werden kann.

## Patentansprüche

1. Übungs- und Untersuchungsgerät für die menschlichen Augen, mit einem an einem Ende eines langgestreckten Gehäuses aufragenden Rahmen, der eine Stirn- und/oder Kinnstütze sowie eine Halterung für zwei optische Gläser trägt, und einer in Längsrichtung des Gehäuses verfahrbaren, im wesentlichen in Höhe der optischen Gläser liegenden Lichtquelle am oberen Ende eines Stabes, der aus einem Schlitz des Gehäuses austritt und durch eine im Gehäuse liegende, durch einen Motor in Drehung versetzbare, in Längsrichtung des Gehäuses verlaufende Spindel antreibbar ist, dadurch gekennzeichnet, daß im oberen Bereich des Gehäuses (10) zwei in dessen Längsrichtung verlaufende Führungsachsen (110,112) vorgesehen sind, auf denen ein Schlitten (114), der den Stab (44) trägt, gleitend verschiebbar ist, und daß der Schlitten (114) an einem schwenkbaren Arm (116) ein Muttersegment (118) trägt, das durch Schwenken des Armes (116) mit der Spindel (106) in Eingriff bringbar ist.

2. Übungs- und Untersuchungsgerät nach Anspruch 1, dadurch gekennzeichnet, daß der Stab (44) an einem Abdeckband (90) befestigt ist, das breiter als der Schlitz (42) ist, und daß zur Führung des Abdeckbandes (90) zwei dessen seitliche Ränder übergreifende Leisten (86,88) auf der Oberseite des Gehäuses (10) befestigt sind.

3. Übungs- und Untersuchungsgerät nach Anspruch 1 oder 2, gekennzeichnet durch eine angrenzend an den Stab (44) vorgesehene Druckplatte (142), die über einen in senkrechter Richtung verschiebbaren Druckstab (140) derart mit dem Muttersegment (118) in Verbindung steht, daß durch Niederdrücken der Druckplatte (142) das Muttersegment aus dem Gewinde der Spindel (106) abhebbar ist.

## Claims

1. Exercise and examination apparatus for human eyes with a frame rising up at one end from a longitudinally extending housing, bearing a forehead and/or chin support as well as a holder for two optical glasses and a light source at the upper end of a bar located substantially at the height of the optical glasses and which can be displaced in the longitudinal direction of the housing, whereby the bar rises from a slot of the housing and can be driven by a spindle which is located in the housing, which runs in the longitudinal direction of the housing and which can be set in rotation by a motor, characterised in that in the upper area of the housing (10) two guide shafts (110, 112), which run in its longitudinal direction are provided, on which a carriage (114), which bears the rod (44) can be slid and in that the carriage (114) bears a nut segment (118) on a swivelling arm (116), which can be brought into engagement with the spindle (106) by swivelling the arm (116).

2. Exercise and examination apparatus according to claim 1, characterised in that the bar (44) is attached to a cover band (90), which is wider than the slot (42) and in that for locating the cover band (90), two strips (86, 88) which overlap its lateral edges are attached to the upper side of the housing (10).

3. Exercise and examination apparatus according to claim 1 or 2, characterised by a pressure plate (142) located so that it is adjacent to the bar (44), whereby the pressure plate is connected to the nut segment (118) via a pressure bar (140) which can be displaced in the vertical direction, in such a way that the nut segment can be lifted out of the thread of the spindle (106) by again pressing the pressure plate (142).

## Revendications

1. Appareil d'exercice et d'examen pour les yeux humains comportant un cadre faisant saillie à une extrémité d'un logement allongé et qui porte un appui pour le front et/ou le menton ainsi qu'un support pour deux verres optiques, et une source de lumière mobile dans le sens longitudinal du logement, disposée sensiblement à hauteur des verres optiques à l'extrémité supérieure d'un barreau qui sort d'une fente du logement et qui est susceptible d'être entraîné par une broche disposée dans le logement, susceptible d'être mise en rotation par un moteur et s'étendant dans le sens longitudinal du logement, caractérisé par le fait que dans la zone supérieure du logement (10) il est prévu deux axes de guidage (110, 112) disposés dans le sens longitudinal de celui-ci, sur lesquels est déplaçable par glissement un chariot (114) qui porte le barreau (44), et que le chariot (114) porte, à un bras pivotant (116) un segment d'écrou (118) qui, par pivotement du bras (116), peut être amené en prise avec la broche (106).

2. Appareil d'exercice et d'examen selon la revendication 1, caractérisé par le fait que le barreau (44) est fixé à une bande de recouvrement (90) qui est plus large que la fente (42) et que, pour guider la bande de recouvrement (90), deux baguettes (86, 88) recouvrant ses bords latéraux sont fixées sur le côté supérieur du logement (10).

3. Appareil d'exercice et d'examen selon la revendication 1 ou 2, caractérisé par une plaque de pression (142) prévue adjacente au barreau (44) qui, par l'intermédiaire d'un barreau de pression (140) mobile verticalement, est en liaison avec le segment d'écrou (118) de manière telle que, par appui vers le bas de la plaque de pression (142) le segment d'écrou peut être soulevé du filetage de la broche (106).

# FIG. 1

FIG. 2

FIG. 3

FIG. 4

0 164 439

FIG. 5

# FIG. 6

FIG. 7